(19) Europäisches Patentamt
European Patent Office
Office européen des brevets

(11) **EP 3 417 768 B1**

(12) **EUROPEAN PATENT SPECIFICATION**

(45) Date of publication and mention
of the grant of the patent:
**17.01.2024 Bulletin 2024/03**

(21) Application number: **18178583.3**

(22) Date of filing: **19.06.2018**

(51) International Patent Classification (IPC):
***A61B 5/00*** *(2006.01)*     ***A61B 5/053*** *(2021.01)*

(52) Cooperative Patent Classification (CPC):
**A61B 5/0536; A61B 5/0093**

(54) **METHOD FOR IMAGING**

BILDGEBUNGSVERFAHREN

PROCÉDÉ D'IMAGERIE

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **19.06.2017 GB 201709774**

(43) Date of publication of application:
**26.12.2018 Bulletin 2018/52**

(73) Proprietor: **Middlesex University Higher
Education Corporation
London NW4 4BT (GB)**

(72) Inventors:
- **BAYFORD, Richard**
  **Surrey, SM2 5LG (GB)**
- **RADEMACHER, Tom**
  **Oxford, OX1 5EY (GB)**
- **ROITT, Ivan**
  **London, NW11 7HB (GB)**
- **DEMOSTHENOUS, Andreas**
  **Herts, HP4 3JJ (GB)**

(74) Representative: **Lucas, Brian Ronald et al
Lucas & Co.
135 Westhall Road
Warlingham, Surrey CR6 9HJ (GB)**

(56) References cited:
**WO-A1-2012/102819**     **WO-A2-2009/045885**
**US-A- 6 122 544**     **US-A1- 2006 058 600**
**US-A1- 2008 095 714**

- C. B. COLLINS ET AL: "Radiofrequency heating pathways for gold nanoparticles", NANOSCALE, vol. 6, no. 15, 1 January 2014 (2014-01-01), pages 8459-8472, XP055522311, United Kingdom ISSN: 2040-3364, DOI: 10.1039/C4NR00464G
- B. BLAD ET AL: "Quantitative assessment of impedance tomography for temperature measurements in hyperthermia", INTERNATIONAL JOURNAL OF HYPERTHERMIA, vol. 8, no. 1, 9 January 1992 (1992-01-09) , pages 33-43, XP55521762, GB ISSN: 0265-6736, DOI: 10.3109/02656739209052877
- M. J. MOSKOWITZ ET AL: "Temperature field estimation using electrical impedance profiling methods. II. Experimental system description and phantom results", INTERNATIONAL JOURNAL OF HYPERTHERMIA, vol. 10, no. 2, 9 January 1994 (1994-01-09), pages 229-245, XP055521594, GB ISSN: 0265-6736, DOI: 10.3109/02656739409009345
- ELBAKRY ASMAA ET AL: "Layer-by-Layer Coated Gold Nanoparticles: Size-Dependent Delivery of DNA into Cells", SMALL, [Online] vol. 8, no. 24, 22 August 2012 (2012-08-22), pages 3847-3856, XP055973299, ISSN: 1613-6810, DOI: 10.1002/smll.201201112 Retrieved from the Internet: URL:https://api.wiley.com/onlinelibrary/td m/v1/articles/10.1002%2Fsmll.201201112> [retrieved on 2022-10-21]

- Wen Jiang ET AL: "Nanoparticle-mediated cellular response is size-dependent", Nature Nanotechnology, vol. 3, no. 3, 1 March 2008 (2008-03-01), pages 145-150, XP055018462, ISSN: 1748-3387, DOI: 10.1038/nnano.2008.30 & Jiang Wen ET AL: "NNANO-0707057B S1 Supplementary Information for: Nanoparticle-mediated cellular response is size-dependent", , 1 March 2008 (2008-03-01), pages S1-S17, XP055973345, Retrieved from the Internet: URL:https://static-content.springer.com/es m/art%3A10.1038%2Fnnano.2008.30/MediaObje c ts/41565_2008_BFnnano200830_MOESM1_ESM. pdf [retrieved on 2022-10-20]
- BAYFORD RICHARD H. ET AL: "Locating Functionalized Gold Nanoparticles Using Electrical Impedance Tomography", IEEE TRANSACTIONS ON BIOMEDICAL ENGINEERING, [Online] vol. 69, no. 1, 18 June 2021 (2021-06-18), pages 494-502, XP055973474, USA ISSN: 0018-9294, DOI: 10.1109/TBME.2021.3100256 Retrieved from the Internet: URL:https://ieeexplore.ieee.org/stampPDF/g etPDF.jsp?tp=&arnumber=9497667&ref=aHR0c HM 6Ly9pZWVleHBsb3JlLmllZWUub3JnL2RvY3VtZ W50L zk0OTc2Njc=> [retrieved on 2022-10-21]
- GRIFFITHS H ET AL: "Applied potential tomography for non-invasive temperature mapping in hyperthermia", CLINICAL PHYSICS AND PHYSIOLOGICAL MEASUREMENT, INSTITUTE OF PHYSICS PUBLISHING, BRISTOL, GB, vol. 8, no. 4A, 1 November 1987 (1987-11-01), pages 147-153, XP020026358, ISSN: 0143-0815, DOI: 10.1088/0143-0815/8/4A/019

## Description

### FIELD OF THE INVENTION

The present invention relates to a method for indicating the location of nanoparticles ('NP') in the body of a mammal. In other aspects the present invention relates to use of electromagnetic radiation or an oscillating magnetic field and electrical impedance tomography to image nanoparticles in or on the human or animal body.

### BACKGROUND TO THE INVENTION

In recent years the use of nanotechnology has advanced considerably in the medical field. Nanotechnology is generally defined as the manipulation of matter on an atomic, molecular, or supramolecular scale in the order of one dimension between about 1nm and about 100 nm. The basis of the 100 nm limit is that novel properties that differentiate particles from the bulk material typically develop at a critical length scale of under 100 nm. Particles having two or three dimensions within this size range are frequently referred to as 'nanoparticles'.

The term nanoparticle does not necessarily give a correct perception of what a nanoparticle is; in particular, there can be a tendency to think of nanoparticles as tiny spherical objects. A better perception of a nanoparticle is comprising a scaffold core formed of atoms on to which other atoms and/or molecules have been attached to make a more complicated structure. For example 57 atoms of gold can be used as a scaffold core on which further atoms can be attached, followed by the addition of biological ligands. Nanoparticles can also be created using different composite materials at the nanoscale, changing their physical properties. As mentioned, these properties do not generally coincide with larger non-nanoscale counterparts.

The material used as the scaffold core in the nanoparticle includes (but is not limited to) atoms of gold, iron, silver, cerium, titanium dioxide, silica, carbon, zinc, copper, nickel, magnesium and composites thereof. Gold is at present preferred in the construction of nanoparticles as it is hydrophobic. Although gold is diamagnetic composites have been produced, for example gold and gold-iron oxide magnetic glyconanoparticles, to combine the advantage of gold and magnetics properties.

To allow nanoparticles to be used in or on the human or animal body it is necessary to improve biocompatibility, for example by providing aqueous solubility and to inhibit nanoparticle aggregation. This is often achieved by means of a coating that is applied to the nanoparticle, for example by attaching organic or other biocompatible molecules to the atomic scaffold. In some cases ligands can be used to bind stabilisers to the nanoparticle. Examples of stabilisers include (but are not limited to) citrate and cellulose.

The addition and configuration of ligands and their interaction with the atoms on the nanoparticle surface plays an important role in determining the physicochemical properties of the nanoparticles and therefore their interaction with the human or animal body and biological material. The physical properties of the nanoparticle can also be changed by attachment of other molecules, including biomolecules and drugs. Such nanoparticles have been called 'functionalised nanoparticles', although in this disclosure the general term 'nanoparticle' will be used to mean a nanoparticle that has been functionalised in this way.

In order to pass through various barriers in the human or animal body, such as the kidney, nanoparticles have two or three dimensions in the size range about 1nm to about 6nm, each dimension measured as a hydrodynamic diameter ('HD'). Other barriers, such as the blood-brain barrier, exclude certain kinds of particle, with less dependence on particle size. For example, some studies have shown that nanoparticles of up to 70nm can pass through the blood-brain barrier, providing they have a suitable coating.

A combination of factors often plays a role in the passage of nanoparticles across these barriers. In the human kidney for example, whether a nanoparticle can pass through the glomerular capillary wall is dependent on its size, charge and shape owing to the unique structure of the glomerular capillary wall. A globular gold nanoparticle with a hydrodynamic diameter < 6 nm can pass through the glomerular capillary wall easily, while it is difficult for a larger one (hydrodynamic diameter > 8 nm) to cross through due to the kidney filtration threshold which is about 5.5 nm. Furthermore, the surface charges of a nanoparticle also play an important role in the kidney filtration: a positively charged NP with an HD of 6-8 nm slightly larger than the kidney filtration threshold ('KFT') can pass the kidney filtration barrier due to the favourable charge interactions with the glomerular capillary wall which is negatively charged, whereas filtration through the kidney is difficult for the negatively charged or neutral NP with an HD of 6-8 nm.

Nanoparticles have been suggested for a range of clinical applications, including as contrast agents, for drug delivery and for treatment or therapy. Nanoparticles may be delivered to the patient by injection, by ingestion or by topical application to the skin for example. As described above, the nanoparticles are constructed to perform a function in the body, for example to reach a particular target in the body such as an organ or a tumour. Once at the target, the nanoparticles may deliver a payload or play a role in some other function such as imaging or therapy. Thus, for example, if the NP is to be targeted to a tumour, cancer biomarkers may be attached to the scaffold core. Alternatively, antibodies to specific bacteria may be attached to the NPs in order to detect sepsis.

Accordingly nanoparticles offer many possibilities for use in the clinical setting. However, one problem is locating nanoparticles in or on the body, i.e. whether they have reached their intended target. To that end, a number of imaging modalities have been researched including magnetic resonance imaging (MRI), positron emission tomography (PET), ultrasound and computer tomography (CT). A summary of the modalities can be found in Bayford, R. et al. 2017 Emerging applications of nanotechnology for diagnosis and therapy of disease (http://iopscience.iop.org/article/10.1088/1361-6579/aa7182/pdf) However, there are a number of problems associated with these modalities, including limitation to certain types of nanoparticle (e.g. MRI), exposure to ionising radiation (PET), exposure to X-ray radiation (CT). A further problem with some modalities is that they can only be performed on tissue or cell samples outside the body.

A particular problem of imaging conductive nanoparticles inside the body is that nanoparticles tend to migrate into cells. Some previous attempts to image conductive nanoparticles in the body using electrical impedance tomography ('EIT') have relied on the assumption that the nanoparticles remain in the extra-cellular space, and are able to affect ionic flow in that space. Those nanoparticles that migrate inside cells can no longer affect the ionic flow, and have no influence on EIT.

WO 2009/045885 (Emilianov et al.) discloses an apparatus, method and system for monitoring and controlling radiation therapy. The system includes a radiative source that emits energy that enters a tissue and is absorbed at or a near a target site in the tissue to heat the tissue; an ultrasound transmitter directed at the target site, wherein the ultrasound transmitter emits ultrasound signals to the tissue that has been heated by the radiative source; an ultrasound receiver directed at the target site, wherein the ultrasound receiver receives ultrasound signals emitted from the ultrasound transmitter and reflected from the tissue that has been heated by the radiative source; and a signal processor that processes the received ultrasound signal to calculate a tissue composition scan or tissue temperature scan.

WO 2012/102819 (McKenna et al.) discloses an energy field and target correlation system that automatically correlates the characteristics of target particles and a living organism to compute the characteristics of an energy field that is applied to a living organism to activate the target particles which are bound to or consumed or taken up by invasive agents in the living organism to produce detectable effects which can be used to treat the invasive agents. The energy field must be crafted to properly control the response and localize the extent of the illumination. The System automatically selects a set of energy field characteristics, including: field type, frequency, field strength, duration, field modulation, repetition frequency, beam size, and focal point. The determined energy field characteristics then are used to activate field generators to generate the desired energy field.

## SUMMARY OF THE INVENTION

The present invention is based on the insight that nanoparticles inside or on a part of the body of a mammal can be stimulated to generate a temperature differential within the part of the body, and this temperature differential is detected by electrical impedance tomography (EIT). The EIT is time difference EIT. This enables estimation of the location or presence of the nanoparticles in the part of the body.

By using EIT to detect a temperature differential the location of nanoparticles can be estimated whether they are outside or inside cells in the part of the body.

We have also discovered that when stimulated the nanoparticles produce a faster rate of heating of cells and tissue, than a rate of heating of the cells and tissue that do not contain nanoparticles. So even if the stimulation causes direct heating of the cells and tissue, the faster rate of heating by the nanoparticles generates a temperature differential that can be imaged by EIT. Thus the location of the nanoparticles is indicated by a 'hotspot' in the EIT image of the part of the body. In some embodiments, the stimulation is by electromagnetic radiation or by an oscillating magnetic field.

According to the invention, there is provided a method of indicating the location of nanoparticles in the body of a mammal comprising cells having an extra-cellular space therebetween and the nanoparticles of a size that tend to migrate into the cells, those nanoparticles that migrate inside the cells no longer able to affect an ionic flow in the extra-cellular space, which method comprises the steps of:

(a) taking reference values of impedance within a part of the body at a first time;
(b) stimulating nanoparticles within the part of the body to produce heat so that there is a temperature differential in said part, wherein the stimulating comprises applying electromagnetic radiation or an oscillating magnetic field comprising a frequency in the range 2.5 GHz to 2.7 GHz to said part, the frequency being the resonant frequency of the nanoparticles such that regions not containing nanoparticles are heated by said stimulation, but one or more regions containing nanoparticles is or are heated at a faster rate by said stimulation, thereby generating said temperature differential and indicating the presence of nanoparticles;
(c) imaging the part using time-difference electrical impedance tomography (td-EIT) at a later second time using the reference values in the td-EIT to determine change in impedance at the later second time, whereby said one or more regions containing nanoparticles and heated at a faster rate by said stimulation, thereby generating said temperature differential, is or are indicated by one or more regions of greatest impedance change; and
(d) producing a difference image (60) showing the change in impedance and the regions of greatest impedance change, thereby indicating the location of nanoparticles in the body of the mammal. By "in the body" it is meant that the nanoparticles may be inside the body, or on the body (e.g. on the skin).

The production of heat by the nanoparticles may be in the form of a disruption to the ion flow in the body part. In this way ions must take a more tortuous path, thereby changing impedance. Subsequent references in this disclosure to heating or temperature change should be understood to include this disruption to ion flow.

According to the disclosure but not claimed, there is also provided a method of indicating the location of nanoparticles in the body of a mammal, which method comprises the steps of:

(a) stimulating nanoparticles within a part of the body to disrupt ion flow so that there is a change of impedance in said part; and
(b) imaging the part using electrical impedance tomography, the location of nanoparticles indicated by one or more region within said part having a greater impedance than other regions of said part.

In one embodiment said other regions not containing nanoparticles are heated or disrupted by said stimulation, but said one or more region containing nanoparticles is heated or disrupted at a faster rate by said stimulation, thereby generating said temperature or impedance differential.

In another embodiment, in step (b) the stimulating comprises applying electromagnetic radiation or an oscillating magnetic field to said part.

Said electromagnetic radiation or oscillating magnetic field comprises a frequency in the Ultra High Frequency (UHF) band has a frequency of 2.5 GHz to 2.7 GHz, e.g. 2.59 GHz to 2.61 GHz; and optionally having a frequency of about 2.599 GHz.

Before step (a) said part of the body may have a temperature that is selected from one of the following: a temperature used in cryosurgery, a normal core body temperature of the mammal, and a temperature used for diathermic or hyperthermic therapy or treatment.

In other embodiments, said temperature differential is up to and including about 0.1 °C and about 70°C in magnitude, and optionally between about 0.1 °C and about 50°C in magnitude.

In some embodiments, said electromagnetic radiation or an oscillating magnetic field has a radiant flux between about 1W and about 100W. By varying the radiant flux it is possible to control that rate at which heat is generated by the nanoparticles.

According to the present disclosure, electrical impedance tomography may comprise absolute electrical impedance tomography (a-EIT) imaging.

According to the invention, however, said electrical impedance tomography comprises time-difference electrical impedance tomography (td-EIT) imaging.

Both a-EIT and td-EIT may employ a single frequency or multi-frequency currents or voltages.

In some aspects, the td-EIT comprises the step of taking reference values of impedance at a first time, and using the reference values in the td-EIT to determine change in impedance at a later second time, whereby said one or more region of greater temperature is indicated by one or more region of greatest impedance change.

In certain embodiments, steps (b) and (c) are performed simultaneously.

In other embodiments, the method further comprises ceasing stimulation of the nanoparticles in step (b) before commencing the electrical impedance tomography imaging of step (c).

In some embodiments, the method further comprises the step of repeating steps (b) and (c) periodically, whereby the location of the nanoparticles is tracked with time. For example the period may be on the scale of minutes or hours.

The nanoparticles may be functionalised nanoparticles in certain embodiments, and may be metallic and/or magnetic, or noble-metal nanoparticles.

In one embodiment the nanoparticles comprise gold atoms, for a scaffold core of gold atoms. In some embodiments the nanoparticles comprise iron atoms, or a composite of gold and iron atoms. In some embodiments the nanoparticles may comprise iron oxide nanoparticles, such as magnetite and maghemite.

In some embodiments, the method further comprises the step of displaying to a user an image of the part of the body that has been imaged by the electrical impedance tomography, in which image said temperature differential is apparent.

In certain embodiments said mammal is a human.

In some embodiments said body part is one of: brain, skin, kidney, liver, lungs, colon, prostate, and cervix.

## BRIEF DESCRIPTION OF THE FIGURES

For a better understanding of the present invention reference will now be made, by way of example only, to the accompanying drawings in which: -

Fig. 1 is a block diagram of an apparatus for demonstrating the principles of the present invention;
Fig. 2 is a graph of temperature versus time for an experiment using the apparatus of Fig. 1;
Fig. 3 is a block diagram of a first embodiment of an apparatus according to the present disclosure;
Fig. 4 is a block diagram of a printed circuit board used in the apparatus of Fig. 3;
Fig. 5A is a first EIT difference image obtained using the apparatus of Fig. 3;
Fig. 5B is a second EIT difference image obtained using the apparatus of Fig. 3;
Fig. 5C is a third EIT difference image obtained using the apparatus of Fig. 3;
Fig. 5D is a fourth EIT difference image obtained using the apparatus of Fig. 3;
Fig. 5E is a fifth EIT difference image obtained using the apparatus of Fig. 3;
Fig. 6 is schematic block diagram of a second embodiment of an apparatus according to the present disclosure; and
Fig. 7 is a graph of temperature increase obtained by stimulating various nanoparticles with radio frequency.

## DETAILED DESCRIPTION

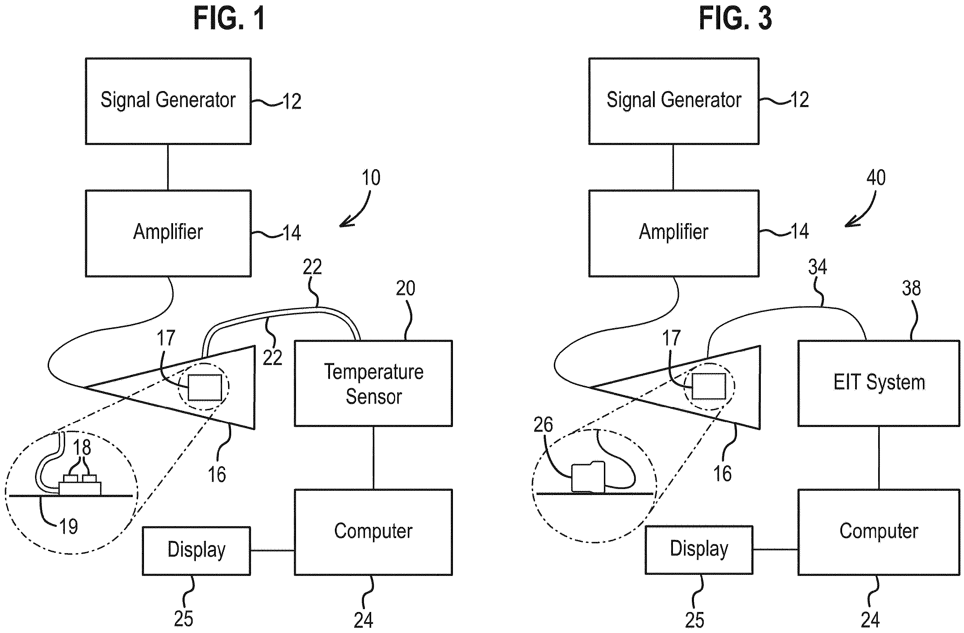

Referring to Fig. 1 an apparatus 10 comprises a signal generator 12 connected to an amplifier 14. An output of the amplifier 14 is connected to an input of a Gigahertz Transverse Electromagnetic (GTEM) cell 16. Access to the interior of the GTEM cell 16 is provided by a door 19 in the side wall. Inside the GTEM cell 16 a shelf holds a pair of microtubes 18. Externally of the GTEM cell 16 a temperature probe 20 has two channels for taking a pair of temperature measurements by optic fibres 22. The temperature probe 20 is connected to a computer 24 to record the temperature measurements with time, which results may be viewed using a display 25 connected to the computer 24.

The signal generator 12 is model 2031 by Marconi Instruments, which can generate a single frequency selectable by the user between 10kHz to 2.7GHz in steps of 1Hz. An alternative signal generator 12 that has been used is a network analyser: an Agilent Technologies model E5701C with a 9kHz to 8.5GHz frequency range. The amplifier 14 is model 5S1G4 by AR Inc. It is a linear amplifier producing 5W over the range 800MHz to 4.2GHz. In another experiment (described below) a different AR amplifier was used which produces 50W over the range 1MHz to 1000MHz. The GTEM cell 16 comprises an enclosure made of a conductive material, such as metal, in the shape of a long, rectangular pyramid. The dimensions of the base of the rectangular pyramid are 0.5m by 0.75m, and its height is 1.25m. The GTEM cell 16 forms an enclosed transverse electromagnetic mode (TEM) stripline. One end of the enclosure is connected to a source of radiation (amplifier 14) and the other end terminated with a 50 Ohm RF load. In operation, emitted radiation travels inside and along the length of the chamber and is absorbed by the RF load at the end.

Each microtube 18 is catalogue number s1615-5500 by Eppendorf AG and can hold 1.5ml of liquid. An integral cover (not shown) allows the liquid to be enclosed inside each microtube. A hole was drilled in each cover to allow a respective optic fibre 22 to reach inside each of the microtubes 18. The temperature probe 20 is a fibre optic thermometer model 812 by Lumasense and has a temperature resolution of 0.01°C. The temperature probe 20 is connected by a data cable to a USB port in the computer 24, which is a desktop PC with a Windows 7 operating system. In use, the temperature probe 20 takes temperature measurements and sends values to the computer 24 for storage.

*-First experiment*

A first experiment was conducted to determine whether any heating effect is caused when nanoparticles in solution are exposed to electromagnetic radiation in the GTEM cell 16.

A first microtube 18 was filled with 1:1 proportion of potassium ferrocyanide - $K_4[Fe(CN)_6]$ - and potassium ferricyanide - $K_3[Fe(CN)_6]$ as a control sample. A second microtube 18 was filled with 20 $\mu$l of an aqueous solution of citrate-stabilised gold nanoparticles. The solution was obtained from Cline Scientific (product code NP01-0051010) and has the following properties:

- Gold nanoparticles of size 5nm $\pm$ 2nm
- CV <15%
- citrate stabilized in "Milli-Q" water
- no surfactants or added stabilizers
- 0.06 mg/ml mass concentration

"Milli-Q" is a trademark of Millipore Corporation to describe "ultrapure" water of Type 1, as defined by various authorities (e.g. ISO 3696), as well as their devices for producing such water. The purification processes involve successive steps of filtration and deionization to achieve a purity characterised in terms of resistivity, typically 18.2 M$\Omega$·cm at 25 °C.

The microtubes 18 were placed on the shelf inside the GTEM cell 16. The tip of one optical fibre 22 of the temperature probe 20 was placed in the water in the first microtube, and the tip of the other optical fibre 22 was placed in the gold nanoparticle solution in the second microtube. The door 19 was closed, and the microtubes 18 left inside the GTEM cell 16 for 30 minutes to allow temperature of the liquids in the wells to equalise with the temperature inside the GTEM cell 16. The temperature inside the room was 19.5 °C at the start of the experiment.

At the end of the time, a frequency of 2.599GHz was selected on the signal generator 12. The signal passed through the amplifier 14 and TEM electromagnetic waves produced inside the GTEM cell 16 at 5W radiant flux. The signal was applied for a time of 60 minutes, and temperature in each well recorded by the computer 24 during that time. At the end of the experiment it was observed that the temperature of the control sample (i.e. without nanoparticles) had increased from 19.50 °C to 20.09 °C, whereas the temperature of the gold nanoparticle solution had increased from 19.50 °C to 20.40°C. The temperature inside the room at the end of the experiment was 19.5 °C.

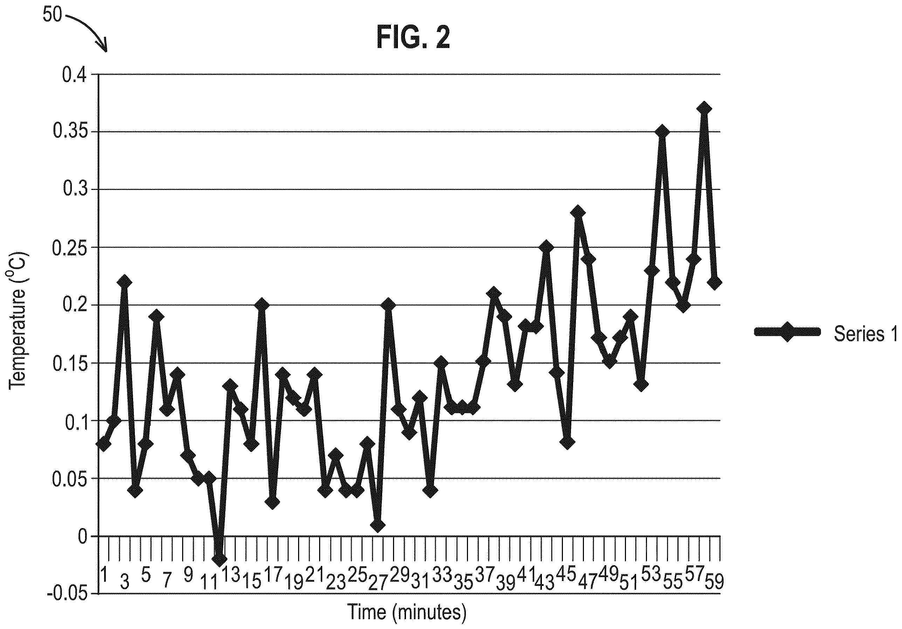

Fig. 2 is graph 50 of temperature (y-axis) versus time (x-axis). For each minute, the temperature value on the graph 50 was generated by subtracting the control sample temperature value from the gold nanoparticle solution temperature value resulting in a temperature difference. The graph illustrates the increasing difference between the temperature of the gold nanoparticle solution and the temperature of the control sample solution. Over the course of the experiment, the temperature difference had risen from between 0.05 - 0.2°C at the beginning to 0.2 - 0.35°C after one hour.

*-Second experiment*

The first experiment was repeated, except that the gold nanoparticle solution was replaced with a different gold nanoparticle solution (0.5mg/ml of 18 nm gold nanoparticles diluted in water). The solution was obtained from Midatech. The control solution was replaced by deionised water.

The alternative AR amplifier (50W over the range 1MHz to 1000MHz) was used to amplify a signal of 12.9MHz from the signal generator 12. The radio-frequency electromagnetic radiation was applied to the samples for a period of 30 minutes. Temperature and EIT imaging were performed in the same way as the first experiment.

Over the course of the experiment, the temperature of the control solution increased by 0.07 °C whereas the temperature of the gold nanoparticle solution increased by 0.18 °C.

It is possible to repeat the first and second experiments with different nanoparticles and with different frequencies to find those that produce a faster rate of heating than the control sample.

*- Third experiment*

Having established that the rate of increase in temperature of the gold nanoparticle solution was greater than the control sample, a third experiment was performed in which an EIT imaging technique was used to examine the two samples whilst being irradiated in the GTEM cell 16.

Biomedical Electrical Impedance Tomography (hereinafter 'EIT') is a non-invasive medical imaging technique in which an image of the conductivity or permittivity of an interior part of the human or animal body is inferred from electrical measurements made by electrodes placed on the skin. In humans, EIT may be used on all patient groups, but has particular promise for those patient groups where other medical imaging techniques (e.g. X-ray, CT scan, MRI scan) are not ethical and/or not possible. One example of the latter is pre-term or full term neonates.

EIT relies upon determination of biological tissue transfer impedance; measurement of the transfer impedance requires placement of electrodes in direct contact with tissues or skin, which enables the injection of a known current from which the resultant voltage can be measured, or vice-versa. Current injection and resultant voltage measurement can be performed through a variety of methods, the most common of which are the two-electrode (bipolar) and four-electrode (tetrapolar) methods. The bipolar method uses the same pair of electrodes to apply current and to measure the resulting voltage. As both current injection and voltage measurement are carried out on the same pair of electrodes, the impedance measured includes not only tissue impedance but also electrode impedance. This can cause inaccuracy if calibration is not performed to find the electrode impedance beforehand. In the tetrapolar method an outer pair of electrodes is used for current injection and an inner pair of electrodes is used to measure voltage. In this way the electrode impedance is eliminated, giving more accurate results.

Different tissue types exhibit different electrical properties (conductivity, resistivity, permittivity, capacitive and impedance) depending on their dimensions, water content, ion concentration, and tissue structure. Measuring bioimpedance can therefore be used to monitor physiological processes, tissue abnormality, tissue characterisation, and can be used to generate a series of images of tissue impedance contrast. The latter can be achieved through taking sets of measurements using a large number of electrodes over a range of frequencies. The images can either be cross-sectional spatial images or a three-dimensional model. The spatial resolution of the images produced is dependent upon the number of measurements taken and the type of electrodes used.

The various methods of generating images based on measurement of tissue electrical properties, such as tissue impedance distribution contrast, are known by different terms: bio-impedance imaging, resistance imaging, impedance tomography, applied potential tomography (APT) and electrical impedance tomography (EIT), the latter including both single-frequency and multi-frequency EIT. EIT is the most commonly used terminology today in the literature and in practice, and will be used throughout this document.

The basic principle of EIT is the mapping of interior impedivity/resistivity distribution of a body region by injecting a small current and measuring the resultant voltage, or vice-versa, through one or more arrays of electrodes attached equidistantly around the surface of the subject. One known method (sometimes called the 'neighbouring' method) for obtaining an impedance image is by injecting a small known current to an adjacent pair of electrodes and measuring the voltage differences generated between other adjacent pairs of electrodes all the way around the subject. A.C. current is used instead of D.C. current because the latter would cause the polarization of ions within the tissues and hence may cause burns on the skin. Typically, the injected A.C. current is very small: international standards limit A.C. current to below 0.1mA rms and 10mA rms for frequencies up to 1 kHz and above 100 kHz respectively, to ensure the safety of subject. Once all possible measurements have been take for the first pair of current injection electrodes, current is injected at the next adjacent pair of electrodes around the subject. Voltage differences are then taken for each adjacent pair of electrodes around the subject. This process is repeated until current has been injected through every adjacent pairs of electrodes.

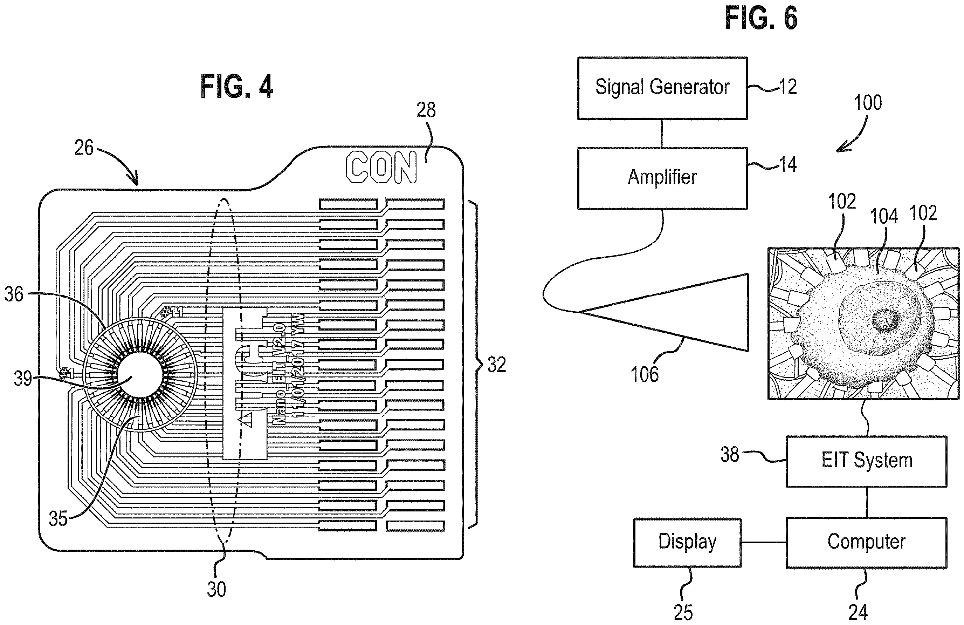

Referring to Fig. 3 an apparatus generally indicated by reference numeral 40 is similar to the apparatus 10 of Fig. 1, with like numerals indicating like parts. However, in the apparatus 40 the microtube 18 was replaced by a printed circuit board ('PCB') 26, shown in greater detail in Fig. 4. The PCB 26 is a miniaturised version of the electrode part of an EIT system that would be used on a patient. The PCB 26 comprises a board 28 onto which are etched thirty-two conductive tracks 30. One end of the conductive tracks 30 terminates along a connection portion 32 of the board 28, to which a ribbon cable 34 can be connected. The other end of the conductive tracks 30 is arranged around a circle 36 of 3.5mm diameter, with each conductive track lying on the radius of the circle 36. These ends are the electrodes 35, and each electrode measures 0.152mm by 1.524mm. An area 39 of 6.35mm diameter within the circle 36 is free of electrodes 35, providing a space for a small volume of sample solution. Solder mask is not applied inside the circle 34 to permit electrical conductivity between the electrodes when the sample solution is in position. It will be noted that in the experiment the PCB 26 was rotated through 90 degrees from the position shown in Fig. 3 so that it laid flat on the shelf 19.

The PCB 26 is connected to a ribbon cable 34 the other end of which is connected to an EIT system 38. The EIT system 38 is a Pioneer Set available from Swisstom AG, comprising an interface with electronics for injecting current between pairs of electrodes, and measuring impedance between other pairs of electrodes (as is in known in the electrical impedance tomography technique).

The Pioneer Set has programmable injection current of 1 to 7 mA peak and injection current frequency 50 kHz-250 kHz AC. Injection patterns can be freely programmed by the user. The load impedance can be up to 1 kQ. Differential and absolute voltage measurements are possible, with between 1 and 80 data frames per second. In this experiment a current of 3mA at 200kHz were used. Data was taken at 50 fps.

The EIT system 38 is connected to the computer 24. The computer 24 is a general purpose PC running the Windows 7 operating system, on which is installed the EIDORS v3.8 and GREIT software (http://eidors3d.sourceforge.net/). EIDORS is a set of open source software algorithms for forward and inverse modelling for EIT. The GREIT algorithm, contained within the EIDORS software, arose from a collaborative project to develop a standardised linear reconstruction algorithm for lung EIT and is now widely used within the research community.

The GREIT algorithm was configured to operate in a normalized difference imaging mode, also known as time difference imaging. EIT normalized difference imaging calculates a change in impedance from a normal-ized change in measurements:

$$( \mathbf{v}_i^2 - \mathbf{v}_i^1 ) / \mathbf{v}_i^1$$

where $v_i^2$ is the $i^{th}$ component of the measurement frame *after* the change, and $v_i^1$ is the $i^{th}$ component of the measurement frame *before* the change. Essentially, a first or reference image ($v_i^1$) is stored and subtracted from each subsequent image ($v_i^2$). The difference is normalised by dividing by the reference image.

The GREIT algorithm was used the reconstruct the images using a 2D model representing the perimeter of the circle 36.

A 20 μl drop of the control sample was placed in the PCB 26 inside the circle 36. The drop formed a meniscus of a size sufficient to cover the area 39 and to cover the end of each of the conductive tracks 30. The PCB 26 was placed inside the GTEM cell 16, making sure that the drop of the control sample was not disturbed. The ribbon cable 34 was routed through the top of the GTEM cell 16 and the free end connected to the EIT system 28. Note that the optic fibres 22 were not used in this experiment. If so, the tip of the sensors would have to be placed in the solution and this would put an insulator within the circle of electrodes, fundamentally altering the EIT image produced.

The PCB 26 was left for a period of 30 minutes in order to reach thermal equilibrium inside the GTEM cell 16. The temperature inside the room at the beginning of the experiment was 19.5 °C. At the end of the time, a frequency of 2.599GHz was selected on the signal generator 12. The signal passed through the amplifier 14 and TEM electromagnetic waves produced inside the GTEM cell 16 at 5W. The signal was applied for a time of 60 minutes, and EIT imaging performed during that time.

At time t = 0s, current is injected by the EIT system 28 and a first set of initial impedance values is recorded between different pairs of electrodes. This first set of impedance values is the reference for each difference image determined during the experiment.

As an example, at t = 8s current is injected between a pair of electrodes and a second set of impedance values is recorded. As described above, the first set of values is subtracted from the second set, and then divided by the first set. This expresses the impedance as a percentage change relative to the initial or reference set of values. The GREIT algorithm then reconstructs an image of the change in impedance across the sample. The image is constructed using a 2D finite element method (FEM) mesh, in which the percentage change in impedance of each element in the FEM mesh is illustrated.

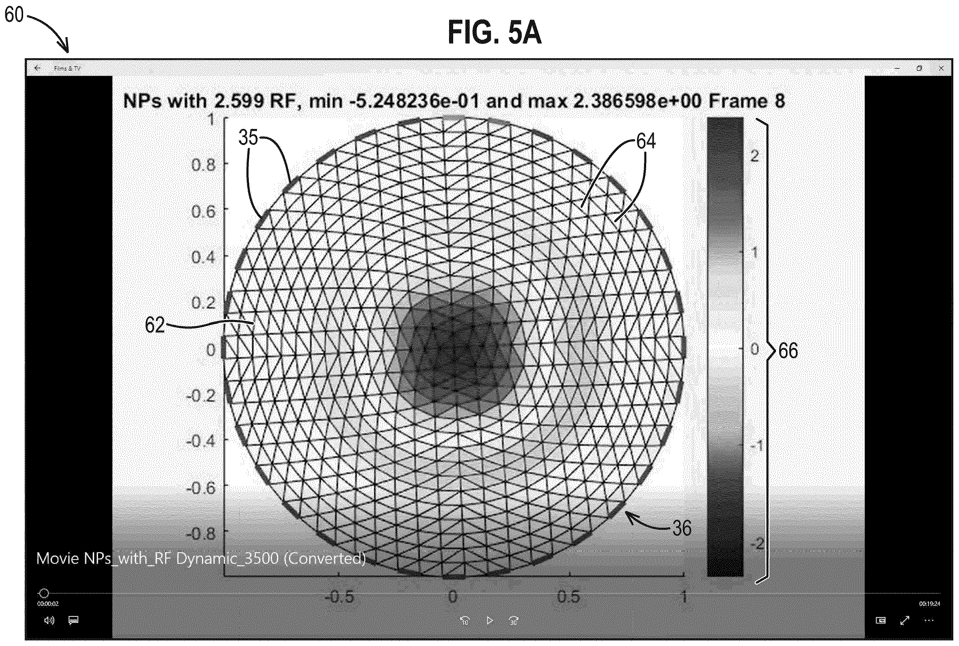

Fig. 5A shows a difference image 60 produced by the GREIT algorithm in EIDORS, from data taken 8s in to the experiment (i.e. frame 8). The difference image 60 shows the circle 36 with the thirty-two electrodes 35 around the circumference, and a finite element (FEM) mesh 62. The GREIT algorithm determines the percentage change in the impedance for each element 64 of the FEM mesh 62 compared to the first set of impedance values taken at t = 0s as described above. The change is shown by different colours on a scale 66. The numbers next to the scale 66 indicate the percentage change in number of hundreds of percent. The scale 66 is auto-adjusted by the software to show the maximum and minimum percent change to be displayed on the FEM mesh 62.

Since impedance increases with temperature, it was expected that as the temperature of the gold nanoparticle solution increased, the change in impedance between the reference data set and the current data set would increase.

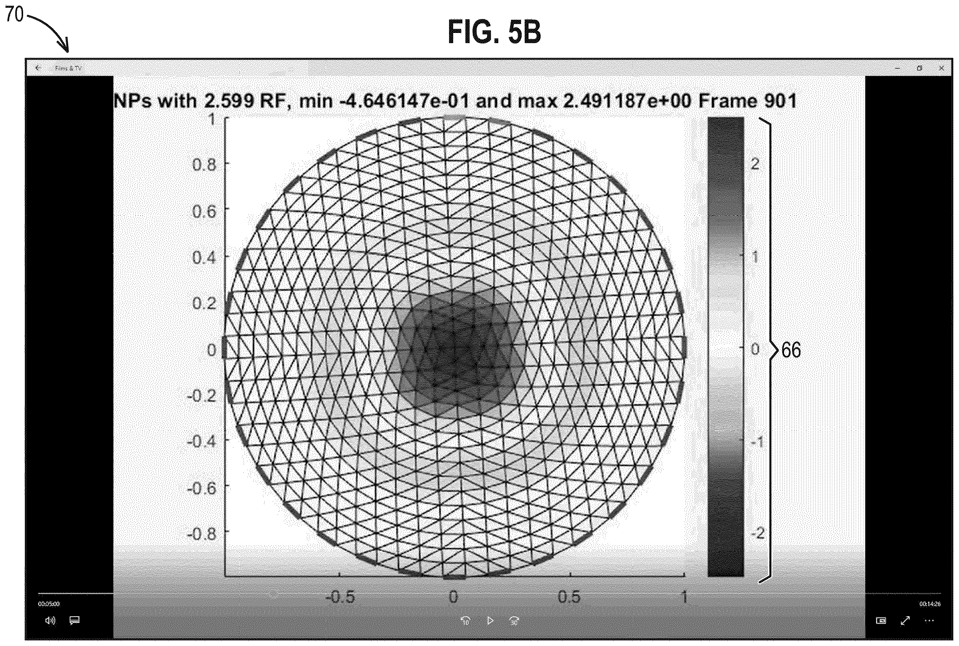

Fig. 5B is a difference image 70 at 15 minutes into the experiment. It is possible to see a small increase in the height of the scale 66, indicating that the maximum impedance to be shown has increased.

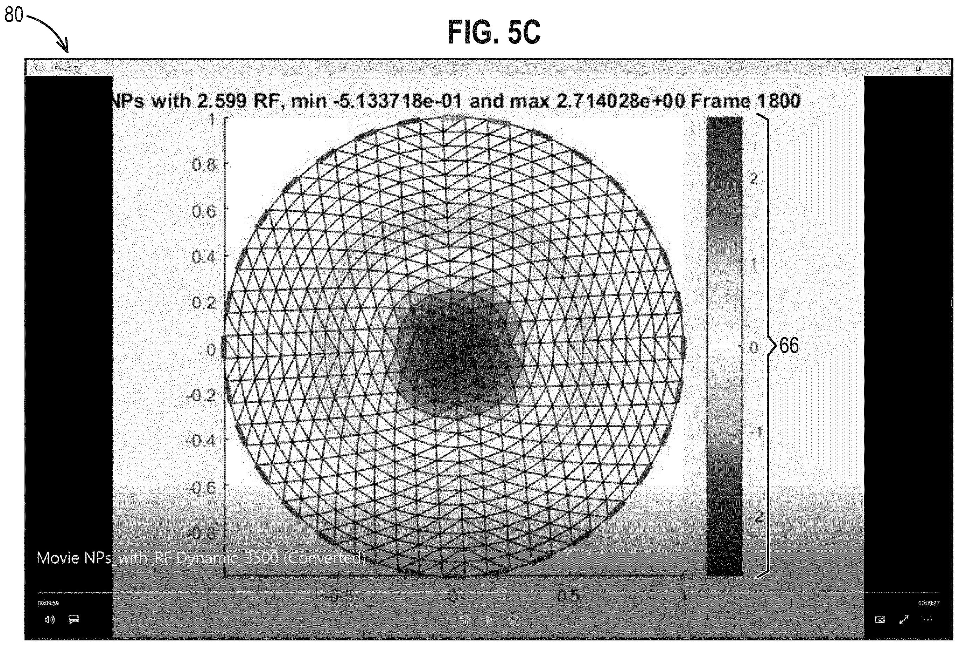

Fig. 5C is a difference image 80 at 30 minutes into the experiment. It is possible to see an increase in the height of the scale 66, indicating that the maximum impedance to be shown has further increased.

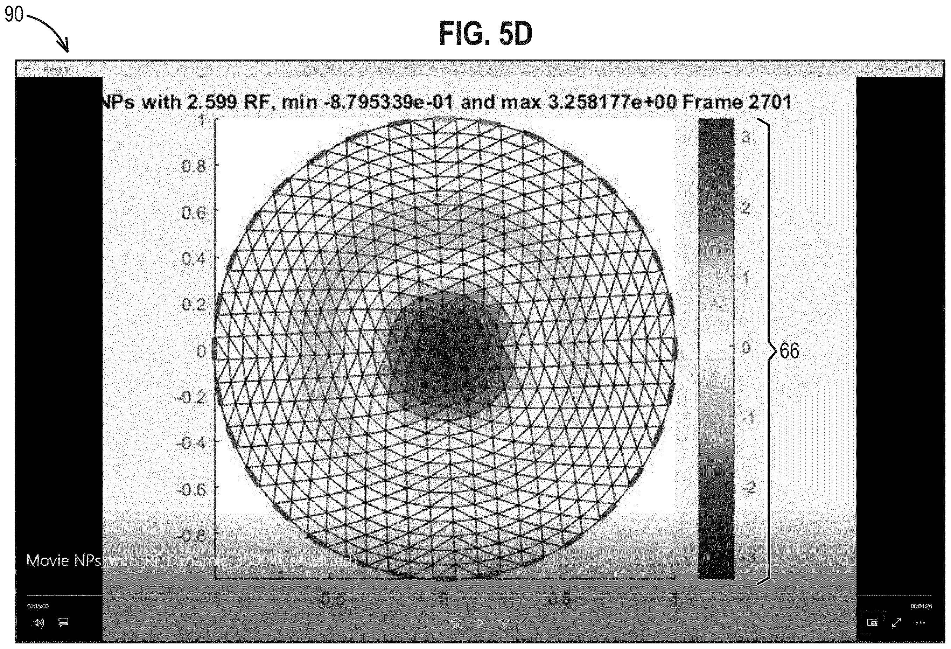

Fig. 5D is a difference image 90 at 45 minutes into the experiment. The number 3 has appeared at the top of the scale 66, indicating that there is a percentage increase in impedance of over 300% over the reference data.

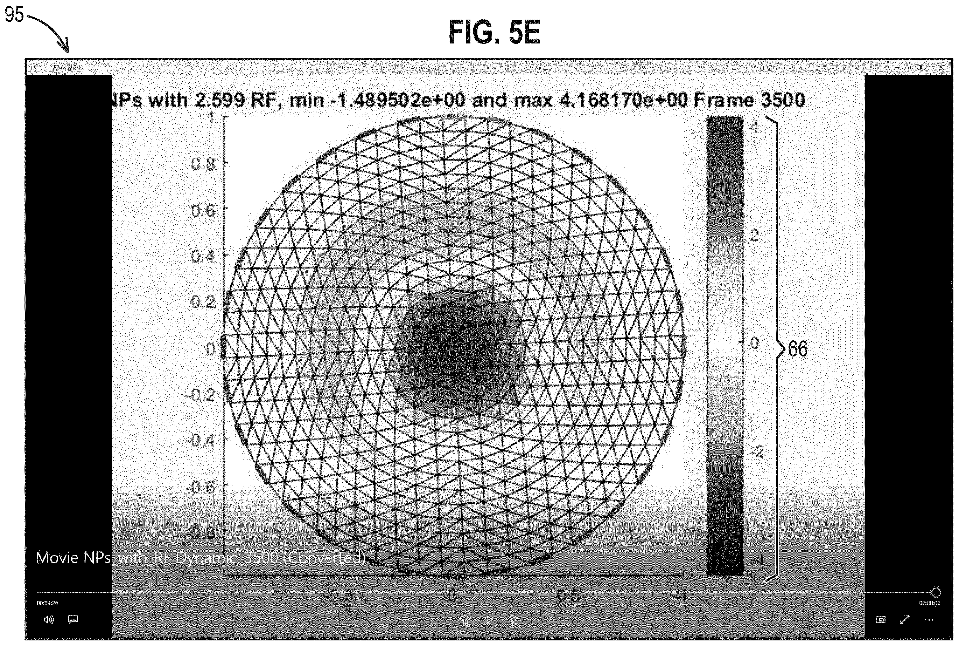

Fig. 5E is a difference image 95 at 58 minutes into the experiment. The number 4 has appeared at the top of the scale 66, indicating that there is a percentage increase in impedance of over 400% over the reference data.

Referring to Fig. 6 an apparatus generally identified by reference numeral 100 is similar to the apparatus 40 of Fig. 3, with like numbers indicating like parts. However, in the apparatus 100 the PCB 26 has been replaced with a set of sixteen electrodes 102 suitable for use with a human breast 104. In this embodiment a patient has been given (e.g. by injection or ingestion) a solution containing nanoparticles to be used as a contrast agent for locating any tumours in the breast 104. For example, the nanoparticles may have been provided with a receptor for binding to a cancer biomarker.

Following delivery of the nanoparticles to the patient, and possibly after a period of time to allow nanoparticles to attach to biomarkers of any tumours in the breast 104, radio-frequency electromagnetic radiation is directed toward the breast 104 by an antenna 106. The antenna is a horn antenna suitable for directing radio-frequency electromagnetic radiation in a beam toward the breast 104. The frequency of the radiation is 2.599 GHz at 5W radiant flux. In this way, any nanoparticles in the breast 104 are stimulated to produce a temperature differential. In particular, wherever the nanoparticles have attached to the cancer biomarker, there will be a localised region having a temperature greater than surround tissue which does not contain nanoparticles. The temperature in the localised region may be between about 0.1 °C and about 70 °C higher than the surrounding tissue.

The production of heat in this way will disrupt the ion flow in the breast tissue where there are nanoparticles, resulting in an impedance change that can be detected by EIT.

*-Electrode pattern*

Although embodiments of the invention have been shown using a circular array of electrodes, it is not limited to this pattern. Other electrode patterns are possible including mats of electrodes or electrodes arranged to as to conform to a surface of the body part, either in 2D or 3D.

The electrodes may also be in the form of a microarray. Such microarrays could be used to locate nanoparticles in or near bundles of neurons in the brain or a nerve for example.

*-Electrode types*

The invention can be used with any electrode used in an EIT system. In some embodiments, the electrodes are active electrodes. In that case, if stimulation and EIT takes place simultaneously it is necessary to shield the amplifiers in the active electrode from the electromagnetic radiation. To that end the use of low input capacitance instrumentation amplifiers in the active electrodes is preferred.

*-Frequency*

In embodiments the frequency or frequencies of the electromagnetic radiation or magnetic field used to stimulate nanoparticles lies in the Ultra High Frequency (UHF) band, i.e. between 2.5 GHz and 2.7 GHz, or 2.59 GHz and 2.61 GHz. In one embodiment the frequency is 2.599 GHz.

Future nanoparticles may be designed to have one or more resonant frequency when stimulated by an electromagnetic field or a magnetic field. Such frequencies may be used to stimulate the nanoparticles to produce a temperature differential for imaging as described herein. Some research has been carried out to devise methods to characterise gold nanoparticles in a way which permits determination of a radio frequency or a band of radio frequencies at which maximum radio frequency absorption occurs. See Nordebo, S. et al. On the physical limitations for radio frequency absorption in gold nanoparticle suspensions, J. Phys. D: Appl. Phys. 50 (2017).

*-Radiant Flux*

Depending on the particular body part to be imaged, the antenna used, and the type of nanoparticles, the radiant power emitted by the antenna may be in the range 1W to 100W in the clinical setting.

*-Duration*

The duration of stimulation of the nanoparticles may be applied over any duration sufficient to cause a temperature differential in a part of the body, the temperature differential being sufficient for detection by EIT. Typically it is expected that it would take no more than a few minutes, e.g. 2-5 minutes, to cause sufficient temperature change depending amongst other things on the radiant power of the antenna, the type of nanoparticles, the body part, etc. Imaging by td-EIT may take place continuously or periodically during the stimulation. One advantage of td-EIT is that the difference image shows impedance (and therefore temperature) change without the need to reference any images from other patients.

Use of absolute EIT is possible, although the image would need to be compared with one or more reference image to determine whether there has been a change of impedance. If a-EIT is use, imaging may take place once either during the stimulation or just after it has ceased, or periodically during stimulation.

*-Temperature differential*

Embodiments of the invention rely on a temperature differential to be generated upon stimulation of the nanoparticles, the temperature differential being sufficient to be discriminated by EIT. At present EIT systems are capable of a distinguishing temperature differences of as little as 0.1 °C.

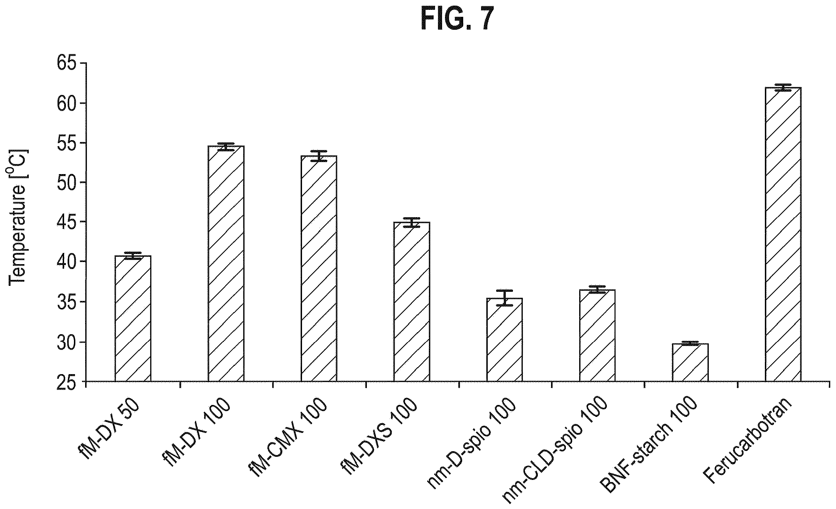

Nanoparticles have been shown to generate various temperature changes upon application of radiofrequencies. Fig. 7 shows temperature increases for various nanoparticles as reported in the thesis Antibody Targeted Magnetic Nanoparticle Hyperthermia for Cancer Therapy, Kozissnik, B., UCL PhD (details at http://discovery.ucl.ac.uk/1415747/). In Fig. 7 the following nanoparticles were from Chemicell GmbH as follows:

- "fm-DX 50": fluidMag-DX dextran coated nanoparticles of 50nm hydrodynamic diameter;
- "fm-DX 100": fluidMag-DX dextran coated nanoparticles of 100nm hydrodynamic diameter;
- "fm-CMX 100": fluidMag-DX carboxymethyldextran coated nanoparticles of 100nm hydrodynamic diameter;
- "fm-DXS 100": fluidMag-DX dextran-sulfate coated nanoparticles of 100nm hydrodynamic diameter;

The following nanoparticles were from micromod Partikeltechnologie GmbH:

- "nm-D-spio 100": nanomag®-D-spio superparamagnetic dextran nanoparticles of 100nm hydrodynamic diameter;
- "nm-CLD-spio 100": nanomag®-D-spio superparamagnetic cross-linked magnetic dextran nanoparticles of 100nm hydrodynamic diameter;
- "BNF starch 100": Bionized NanoFerrite particles coated with hydroxyethyl starch and 100 nm hydrodynamic diameter.

In view of the temperature changes that such nanoparticles can be stimulated to generate, the invention is applicable to a range of temperature differentials caused by stimulation of nanoparticles, from about 0.1 °C to at least about 70 °C.

*- Body part temperature*

In principle, the body part could be at any known body part temperature when stimulation is applied to the nanoparticles. Thus embodiments may find use in therapy and treatment techniques including but not limited to cryosurgery, diathermia, hyperthermia, and ablation. For example it may be necessary to know if nanoparticles have reached or remain within a part of the body during the course of a particular treatment (the recited treatments by therapy or surgery are not according to and are not part of the claimed invention).

In some embodiments, the body part is at or near normal core body temperature. Normal core body temperature of a human lies in the range 36.5 - 37.5 °C. However,

The following table lists some examples of mammals and their core temperature:

| Species | °C | °F |
|---|---|---|
| Beef cow | 36.7-39.1 | 98.0-102.4 |
| Dairy cow | 38.0-39.3 | 100.4-102.8 |
| Cat | 38.1-39.2 | 100.5-102.5 |
| Chicken (daylight) | 40.6-43.0 | 105.0-109.4 |
| Dog | 37.9-39.9 | 100.2-103.8 |
| Goat | 38.5-39.7 | 101.3-103.5 |
| Horse | | |
| Mare | 37.3-38.2 | 99.1-100.8 |
| Stallion | 37.2-38.1 | 99.0-100.6 |
| Pig | 38.7-39.8 | 101.6-103.6 |
| Rabbit | 38.6-40.1 | 101.5-104.2 |
| Sheep | 38.3-39.9 | 100.9-103.8 |

*-Nanoparticles*

The present invention will find use with any nanoparticle that can be stimulated to produce heat, and thereby a temperature differential, in the body of a mammal.

As mentioned above, nanoparticles are self-assembled onto an atomic scaffold core. Such a scaffold core may have a mean diameter between 0.5 and 10 nm, preferably between 1 and 2.5nm. However, the scaffold may not be circular but rather like a lozenge in shape.

The ligands may be covalently linked to the nanoparticle scaffold core and may be selected from carbohydrates, peptides, therapeutic agents such as anticancer or cytotoxic drugs and antibody molecules or fragments thereof which are capable of targeting a tumour by attaching to a specific tumour biomarker, or infectious microbial agents such as bacteria or the like.

The nanoparticles may be produced by conjugating the ligands with the scaffold core of the nanoparticle by derivatizing the ligand with a linker and including the derivatized ligand in a reaction mixture from which the scaffold of the nanoparticle is synthesised. During self-assembly of the nanoparticles, the NP scaffold core attaches to the linker via a linker. The linker may comprise a thiol group, and a C2 to C12 alkyl group, or C2 to C12 glycol group or a peptide group. An example of a thiol group linker is represented by the general formula HO - $(CH_2)_n$ - S - S - $(CH_2)_m$ - OH wherein n and m are independently between 1 and 5. When the nanoparticles are synthesised, the - S - S - of the linker splits to form two thio linkers that can each covalently attach to the scaffold of the nanoparticle via a - S - group. Ligands such as carbohydrates, peptides, antibody molecules and fragments thereof, and therapeutic agents, such as anticancer or cytotoxic drugs, may all be linked simultaneously or in pre-selected combinations to the scaffold of the nanoparticles.

When the ligands are conjugated to the nanoparticle scaffold with a linker, the mean diameter of the nanoparticle including ligands may be from 5nm to 100 nm, 5nm to 70nm, preferably from 10 nm to 50 nm, most preferably from 15 nm to 30nm.

In one embodiment, nanoparticles having scaffolds comprising gold atoms may be synthesised using the protocol first described in WO 02/32404 in which disulphide linkers are employed to derivatize the ligands and the derivatized ligands are reacted with $HAuCl_4$ (tetrachloroauric acid) in the presence of reducing agent to produce the nanoparticles. In this method the disulphide protected ligand in methanol or water may be added to an aqueous solution of tetrachloroauric acid. A preferred reducing agent is sodium borohydride. These and other features of the method are described in WO 02/32404.

*-Meaning of "nanoparticles"*

Several standards setting organisations have tried to define terms in relation to the nanoscale. For example, ISO/TS 27687 lists various terms and definitions related to particles in the area of nanotechnologies. The definition of 'nanoscale' is of a size range from approximately from 1 nm to 100 nm. This definition is accompanied by two notes: (1) properties that are not extrapolations from a larger size will typically, but not exclusively, be exhibited in this size range. For such properties the size limits are considered approximate. (2) the lower limit in this definition (approximately 1 nm) is introduced to avoid single and small groups of atoms from being designated as nano-objects or elements of nanostructures, which might be implied by the absence of a lower limit.

ASTM E2456-06 defines several terms including nanoparticle, fine particle, ultrafine particle, and many others. The ASTM definition of nanoparticle is also essentially 1-100nm, but mentions the number of dimensions and carries a note entitled "Discussion":

"nanoparticle, n-in nanotechnology, a sub-classification of ultrafine particle with lengths in two or three dimensions greater than 0.001 micrometer (1 nanometer) and smaller than about 0.1 micrometer (100 nanometers) and which may or may not exhibit a size-related intensive property.

DISCUSSION-This term is a subject of controversy regarding the size range and the presence of a size-related property. Current usage emphasizes size and not properties in the definition. The length scale may be a hydrodynamic diameter or a geometric length appropriate to the intended use of the nanoparticle."

*-Applications*

It is envisaged that embodiments of the invention may find application in, but not limited to, cancer imaging and treatment, drug discovery (for example drug vectorization), lung monitoring and neuron imaging, these treatments by therapy and/or surgery however not being according to and not being part of the claimed invention.

**Claims**

1. A method of indicating the location of nanoparticles in the body of a mammal comprising cells having an extra-cellular space therebetween and the nanoparticles of a size that tend to migrate into the cells, those nanoparticles that migrate inside the cells no longer able to affect an ionic flow in the extra-cellular space, which method comprises the steps of:

   (a) taking reference values of impedance within a part of the body at a first time;
   (b) stimulating nanoparticles within the part of the body to produce heat so that there is a temperature differential in said part, wherein the stimulating comprises applying electromagnetic radiation or an oscillating magnetic field comprising a frequency in the range 2.5 GHz to 2.7 GHz to said part, the frequency being the resonant frequency of the nanoparticles such that regions not containing nanoparticles are heated by said stimulation, but one or more regions containing nanoparticles is or are heated at a faster rate by said stimulation, thereby generating said temperature differential and indicating the presence of nanoparticles;
   (c) imaging the part using time-difference electrical impedance tomography (td-EIT) at a later second time using the reference values in the td-EIT to determine change in impedance at the later second time, whereby said one or more regions containing nanoparticles and heated at a faster rate by said stimulation, thereby generating said temperature differential, is or are indicated by one or more regions of greatest impedance change; and

(d) producing a difference image (60) showing the change in impedance and the regions of greatest impedance change, thereby indicating the location of nanoparticles in the body of the mammal.

2. A method according to claim 1, wherein said electromagnetic radiation or oscillating magnetic field comprises a frequency of 2.599 GHz.

3. A method according to any preceding claim, wherein said temperature differential is up to and including 0.1°C and 70°C in magnitude, and optionally between 0.1°C and 50°C in magnitude, and optionally wherein said electromagnetic radiation or magnetic field has a radiant flux between about 1W and about 100W.

4. A method according to any preceding claim, wherein either steps (b) and (c) are performed simultaneously, or the method further comprises ceasing stimulation of the nanoparticles in step (b) before commencing the electrical impedance tomography imaging of step (c), the method optionally further comprising the step of repeating steps (b) and (c) periodically, whereby the location of the nanoparticles is tracked with time.

5. A method according to any preceding claim, wherein the nanoparticles are functionalised nanoparticles, optionally conductive and/or metallic and/or magnetic nanoparticles, optionally noble-metal nanoparticles, and optionally comprise gold atoms, iron atoms, a composite of gold and iron atoms, or iron oxide nanoparticles.

6. A method according to any preceding claim, further comprising the step of displaying to a user the difference image.

**Patentansprüche**

1. Verfahren zum Angeben der Position von Nanopartikeln im Körper eines Säugetiers, der Zellen mit dazwischen einem extrazellulären Raum und die Nanopartikeln in einer Größe, die zu einer Migration in die Zellen neigt, umfasst, wobei die in die Zellen migrierenden Nanopartikeln einen Ionenfluss in dem extrazellulären Raum nicht mehr beeinflussen können, wobei die Verfahren die folgenden Schritte umfassen:

(a) Erfassen von Referenzwerten der Impedanz in einem Teil des Körpers zu einer ersten Zeit,
(b) Stimulieren von Nanopartikeln in dem Teil des Körpers für das Erzeugen von Wärme, sodass eine Temperaturdifferenz in dem Teil vorhanden ist, wobei das Stimulieren das Anwenden einer elektromagnetischen Strahlung oder eines oszillierenden Magnetfelds mit einer Frequenz im Bereich von 2,5 GHz bis 2,7 GHz auf den Teil umfasst, wobei die Frequenz die Resonanzfrequenz der Nanopartikeln ist, sodass Bereiche, die keine Nanopartikeln enthalten, durch die Stimulation erwärmt werden, während ein oder mehrere Bereiche, die Nanopartikeln enthalten, mit einer schnelleren Rate durch diese Stimulation erwärmt werden, wodurch eine Temperaturdifferenz erzeugt wird und das Vorhandensein von Nanopartikeln angegeben wird,
(c) Abbilden des Teils unter Verwendung einer Zeitdifferenz-Elektrische-Impedanz-Tomografie (td-EIT) zu einer späteren zweiten Zeit unter Verwendung der Referenzwerte in der td-EIT für das Bestimmen einer Änderung der Impedanz zu der späteren zweiten Zeit, wobei der eine oder die mehreren Bereiche, die Nanopartikeln enthalten und mit einer schnelleren Rate durch die Stimulation erwärmt werden, sodass eine Temperaturdifferenz erzeugt wird, durch einen oder mehrere Bereiche der größten Impedanzänderung angegeben werden, und
(d) Erzeugen eines Differenzbilds (60), das die Änderung der Impedanz und die Bereiche der größten Impedanzänderung zeigt, um die Position von Nanopartikeln in dem Körper des Säugetiers anzugeben.

2. Verfahren nach Anspruch 1, wobei die elektromagnetische Strahlung oder das oszillierende Magnetfeld eine Frequenz von 2,599 GHz aufweist.

3. Verfahren nach einem der vorstehenden Ansprüche, wobei die Temperaturdifferenz eine Größe zwischen 0,1°C und 70°C und optional zwischen 0,1°C und 50°C aufweist und wobei optional die elektromagnetische Strahlung oder das Magnetfeld einen Strahlungsfluss zwischen ungefähr 1 W und ungefähr 100 W aufweist.

4. Verfahren nach einem der vorstehenden Ansprüche, wobei entweder die Schritte (b) und (c) gleichzeitig durchgeführt werden oder das Verfahren weiterhin das Beenden der Stimulation der Nanopartikeln in dem Schritt (b) vor dem Beginnen der Elektrische-Impedanz-Tomografie-Bildgebung des Schritts (c) umfasst, wobei das Verfahren optional weiterhin einen Schritt zum periodischen Wiederholen der Schritte (b) und (c) umfasst und die Position der Nanopartikeln über die Zeit verfolgt wird.

**5.** Verfahren nach einem der vorstehenden Ansprüche, wobei die Nanopartikeln funktionalisierte Nanopartikeln und optional leitende und/oder metallische und/oder magnetische Nanopartikeln wie optional Edelmetall-Nanopartikeln sind und optional Goldatome, Eisenatome, eine Zusammensetzung aus Gold- und Eisenatomen oder Eisenoxid umfassen.

**6.** Verfahren nach einem der vorstehenden Ansprüche, das weiterhin einen Schritt zum Anzeigen des Differenzbilds für einen Benutzer umfasst.

**Revendications**

**1.** Procédé pour indiquer l'emplacement de nanoparticules dans le corps d'un mammifère comprenant des cellules ayant un espace extracellulaire entre elles et les nanoparticules d'une taille qui tendent à migrer dans les cellules, ces nanoparticules qui migrent au sein des cellules ne pouvant plus affecter un flux ionique dans l'espace extracellulaire, lequel procédé comprend les étapes de :

(a) prise de valeurs de référence d'impédance au sein d'une partie du corps à un premier moment ;
(b) stimulation de nanoparticules au sein de la partie du corps pour produire de la chaleur de sorte qu'il y ait un différentiel de température dans ladite partie, dans lequel la stimulation comprend l'application d'un rayonnement électromagnétique ou d'un champ magnétique oscillant comprenant une fréquence dans la plage de 2,5 GHz à 2,7 GHz à ladite partie, la fréquence étant la fréquence de résonance des nanoparticules de telle sorte que des régions ne contenant pas de nanoparticules sont chauffées par ladite stimulation, mais qu'une ou plusieurs régions contenant des nanoparticules est ou sont chauffées à une viLesse plus rapide par ladite stimulation, générant ainsi ledit différentiel de température et indiquant la présence de nanoparticules ;
(c) imagerie de la partie à l'aide d'une tomographie d'impédance électrique à différence de temps (td-EIT) à un second moment ultérieur en utilisant les valeurs de référence dans la td-EIT pour déterminer un changement d'impédance au second moment ultérieur, moyennant quoi lesdites une ou plusieurs régions contenant des nanoparticules et chauffées à une vitesse plus rapide par ladite stimulation, générant ainsi ledit différentiel de température, est ou sont indiquées par une ou plusieurs régions de plus grand changement d'impédance ; et
(d) production d'une image différentielle (60) montrant le changement d'impédance et les régions de plus grand changement d'impédance, indiquant ainsi l'emplacement de nanoparticules dans le corps du mammifère.

**2.** Procédé selon la revendication 1, dans lequel ledit rayonnement électromagnétique ou champ magnétique oscillant comprend une fréquence de 2,599 GHz.

**3.** Procédé selon une quelconque revendication précédente, dans lequel ledit différentiel de température va jusqu'à et incluant 0,1°C et 70°C en grandeur, et facultativement entre 0,1°C et 50°C en grandeur, et facultativement dans lequel ledit rayonnement électromagnétique ou champ magnétique a un flux radiant entre environ 1 W et environ 100 W.

**4.** Procédé selon une quelconque revendication précédente, dans lequel soit les étapes (b) et (c) sont effectuées simultanément, soit le procédé comprend en outre l'arrêt de la stimulation des nanoparticules à l'étape (b) avant de commencer l'imagerie par tomographie d'impédance électrique de l'étape (c), le procédé comprenant en outre facultativement l'étape de répétition périodique des étapes (b) et (c), moyennant quoi l'emplacement des nanoparticules est suivi dans le temps.

**5.** Procédé selon une quelconque revendication précédente, dans lequel les nanoparticules sont des nanoparticules fonctionnalisées, facultativement des nanoparticules conductrices et/ou métalliques et/ou magnétiques, facultativement des nanoparticules de métaux nobles, et comprennent facultativement des atomes d'or, des atomes de fer, un composite d'atomes d'or et de fer, ou des nanoparticules d'oxyde de fer.

**6.** Procédé selon une quelconque revendication précédente, comprenant en outre l'étape d'affichage à un utilisateur de l'image différentielle.

**FIG. 3**

Signal Generator — 12

Amplifier — 14

40

EIT System — 38

34

17

16

Computer — 24

Display — 25

26

**FIG. 1**

Signal Generator — 12

Amplifier — 14

10

Temperature Sensor — 20

22

22

17

16

Computer — 24

Display — 25

18

19

FIG. 2

# FIG. 4

# FIG. 6

EP 3 417 768 B1

**FIG. 5A**

60

NPs with 2.599 RF, min -5.248236e-01 and max 2.386598e+00 Frame 8

Movie NPs_with_RF Dynamic_3500 (Converted)

EP 3 417 768 B1

FIG. 5B

FIG. 5C

## FIG. 5D

90

EP 3 417 768 B1

FIG. 5E

Movie NPs_with_RF Dynamic_3500 (Converted)



FIG. 5E

# FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009045885 A, Emilianov
- WO 2012102819 A, McKenna
- WO 0232404 A

**Non-patent literature cited in the description**

- **BAYFORD, R. et al.** *Emerging applications of nanotechnology for diagnosis and therapy of disease,* 2017, http://iopscience.iop.org/article/10.1088/1361-6579/aa7182/pdf
- **NORDEBO, S. et al.** On the physical limitations for radio frequency absorption in gold nanoparticle suspensions. *J. Phys. D: Appl. Phys.,* 2017, 50